(19) Europäisches Patentamt
European Patent Office
Office européen des brevets

(11) Veröffentlichungsnummer: **0 207 431**
**B1**

(12) **EUROPÄISCHE PATENTSCHRIFT**

(45) Veröffentlichungstag der Patentschrift: **16.08.90**

(51) Int. Cl.$^5$: **C 07 D 265/30,** A 01 N 43/84

(21) Anmeldenummer: **86108627.0**

(22) Anmeldetag: **25.06.86**

(54) **N-Alkyl-2,6-dimethylmorpholinio-carbonsäureamid-Salze, Verfahren zu ihrer Herstellung und ihre Verwendung als Fungizide.**

(30) Priorität: **05.07.85 DD 278324**

(43) Veröffentlichungstag der Anmeldung:
**07.01.87 Patentblatt 87/02**

(45) Bekanntmachung des Hinweises auf die Patenterteilung:
**16.08.90 Patentblatt 90/33**

(84) Benannte Vertragsstaaten:
**AT BE CH DE FR GB IT LI NL SE**

(56) Entgegenhaltungen:
**DE-A-2 657 728**
**DE-A-2 915 250**

**CHEMICAL ABSTRACTS, Band 104, Nr. 17, 28. April 1986, Seite 547, Zusammenfassung Nr. 147137z, Columbus, Ohio, US;**

**Die Akte enthält technische Angaben, die nach dem Eingang der Anmeldung eingereicht wurden und die nicht in dieser Patentschrift enthalten sind.**

(73) Patentinhaber: **Institut für Pflanzenschutzforschung Kleinmachnow der A.d.L. der DDR**
**Stahnsdorfer Damm 81**
**DDR-1532 Kleinmachnow (DD)**

(72) Erfinder: **Banasiak, Lothar, Dr.**
**Albert-Klink-Strasse 24**
**DDR-1500 Potsdam (DD)**
Erfinder: **Edlich, Wilfried, Dr.**
**Strasse der Gemeinschaft 13**
**DDR-1500 Potsdam (DD)**
Erfinder: **Lyr, Horst, Prof. Dr.**
**Georg-Herwegh-Strasse 8**
**DDR-1300 Eberswalde (DD)**
Erfinder: **Nega, Eva**
**Kepler-Platz 4/18**
**DDR-1502 Potsdam-Babelsberg (DD)**
Erfinder: **Sunkel, Marianne**
**Galileistrasse 83**
**DDR-1502 Potsdam-Babelsberg (DD)**

(74) Vertreter: **Grussdorf, Jürgen, Dr. et al**
**Patentanwälte Zellentin & Partner**
**Rubensstrasse 30**
**D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 207 431 B1**

**Beschreibung**

Die Erfindung betrifft neue N-Alkyl-2,6-dimethylmorpholinio-carbonsäureamid-Salze, ihre Herstellung und ihre Verwendung als fungizide Mittel in der Landwirtschaft und in Gartenbau mit zusätzlichen pflanzenwaschstumsregulierenden Eigenschaften.

Es ist bekannt, N-Alkylmorpholine und ihre Salze sowie ihre Molekül- und Additionsverbindungen als Fungizide zu verwenden (DE—PS 1 164 152, DE—PS 1 173 722, DE—PS 24 61 513).

Bekannt ist ferner, daß quaternäre Ammoniumverbindungen von langkettigen N-Alkyl-2,6-dimethylmorpholinen mit niederen Alkyl-, Alkenyl-, Alkoxyalkyl- oder Aralkyl-Substituenten fungizid wirksam sind (DE—PS 1 167 588; Angewandte Chemie 77 (1965), S. 327—333). Ferner sind Mittel bekannt, die substituierte N-Benzyl- oder Alkoxymethyl-2,6-dimethylorpholinium-Salze als Wirkstoffe zur Bekämpfung von pilzlichen Schaderregern enthalten (DD 134 037, DD 134 474, DD 140 403). Es ist weiterhin bekannt, Morpholino-carbonsäureanilide zur Bekämpfung phytopathogener Pilze zu verwenden (DD 141 778).

Desweiteren werden Salze von α-Aminoacetaniliden, die ais Aminkomponente auch den Morpholinring einschließen, mit niederen Alkyl-, Cyanoalkyl-, Alkenyl- und Alkinyl-Substituenten als Mittel zur Beeinflussung bzw. Regulierung des Pflanzenwachstums genannt (DE—PS 26 57 728 und DE—PS 2915 250).

Ferner ist bereits bekannt, 2,3-Dihydro-6-methyl-5-phenylcarbamoyl-1,4-oxathiin-4,4-dioxid (Oxycarboxin), N,N'-Bis-(1-formamido-2,2,2-trichlorethyl)-piperazin (Triforine) oder Zink-ethylen-bis-dithiocarbamat (Zineb) als Wirkstoffe in fungiziden Mitteln zur Bekämpfung von pilzlichen Pflanzenkrankheiten zu verwenden (The Pesticide Manual, British Crop protection Council; London, 1984).

Die Wirkung der genannten Verbindungen ist jedoch in bestimmten Indikationsbereichen, insbesondere bei niedrigen Aufwandmengen und -konzentrationen, nicht immer voll befriedigend. Außerendem zeigen sie eine recht hohe Selektivität gegenüber bestimmten Arten von pilzlichen Schaderregern, wodurch eine breite Anwendung dieser Mittel stark eingeschränkt wird. Nachteilig ist weiterhin, daß die Pflanzenventräglichkeit dieser Verbindungen in vielen Fällen nicht ausreichend ist.

Das Ziel der Erfindung ist die Entwicklung von neuen Verbindungen mit verbesserter fungizider Wirksamkeit und zusätzlichen wachstrumsregulierenden Eigenschaften, ein Verfahren zur deren Herstellung sowie die Verwendung solcher fungiziden Mittel in der Landwirtschaft und im Gartenbau.

Der Erfindung liegt die Aufgabe zugrunde, neue N-Alkyl-2,6-dimethylmorpholinio-carbonsäureamid-Salze und sie enthaltende fungizide Mittel mit guter Wirksamkeit und breitem Wirkungsspektrum sowie einer möglichst hohen Pflanzenverträglichkeit und zusätzlichen pflanzenwachstrumsregulierenden Eigenschaften bereitzustellen. Zur Lösung dieser Aufgabe werden Verbindungen vorgeschlagen, die dadurch gekennzeichnet sind, daß sie mindestens ein N-Alkyl-2,6-dimethylmorpholinio-carbonsäureamid-Salz der allgemeinen Formel I,

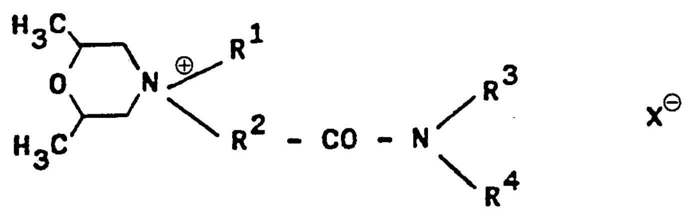

(I)

in welcher

$R^1$ geradkettiges oder verzweigtes Alkyl mit 6 bis 20 C-Atomen,

$R^2$ geradkettiges oder verzweigtes Alkylen mit 1 bis 6 C-Atomen,

$R^3$ und $R^4$ unabhängig voneinander gleich oder verschieden Wasserstoff, geradkettiges oder verzweigtes Alkyl mit 1 bis 20 C-Atomen, ein durch Hydroxy, Alkoxy mit 1 bis 4 C-Atomen, Cyano oder Dialkylamino mit 2 bis 16 C-Atomen substituiertes geradkettiges oder verzweigtes Alkyl mit 1 bis 6 C-Atomen, ein gegebenenfalls durch Halogen substituiertes Alkenyl mit 3 bis 6 C-Atomen, Cycloalkyl mit 3 bis 7 C-Atomen, Aryl, welches einfach oder mehrfach, gleich oder verschieden durch geradkettiges oder verzweigtes Alkyl mit 1 bis 6 C-Atomen, Alkoxy mit 1 bis 4 C-Atomen, Aryl-niedenalkyl mit 7 bis 12 C-Atomen, Cycloalkyl mit 3 bis 7 C-Atomen, Acyl mit 1 bis 4 C-Atomen, Aryl, Halogen, Halogenalkyl mit 1 bis 4 C-Atomen, Halogenalkoxy mit 1 bis 4 C-Atomen, Alkylthio mit 1 bis 4 C-Atomen, Halogenalkylthio mit 1 bis 4 C-Atomen, Nitro, Cyano, Thiocyanato, NHCOR', NHCONR'R'', COOR', CONR'R'', $SO_2R'$ und/oder $SO_2NR'R''$, wobei R' und R'' unabhängig voneinander für Wasserstoff, geradkettiges oder verzweigtes Alkyl mit 1 bis 6 C-Atomen, Cycloalkyl mit 3 bis 7 C-Atomen, Aryl oder Aryl-nieder-Alkyl mit 7 bis 12 C-Atomen steht, substituiert sein kann, Aryl-nieder-alkyl mit 7 bis 12 C-Atomen, welches einfach oder mehrfach, gleich oder verschieden durch geradkettiges oder verzweigtes Alkyl mit 1 bis 6 C-Atomen, Alkoxy mit 1 bis 4 C-Atomen, Halogen, Halogenalkyl mit 1 bis 4 C-Atomen, Nitro und/oder Cyano substituiert sein kann,

$R^3$ und $R^4$ zusammen mit dem Stickstoffatom einen gegebenenfalls ein- oder zweifach durch Alkyl mit 1 bis 4 C-Atomen und/oder Halogen substituierten 3 oder fünf- oder sechsgliedrigen heterocyclischen Ring darstellt, der gegebenenfalls noch ein oder zwei weitere Heteroatome enthält, und

$X^-$ das Anion einer nicht phytotoxischen Säure bedeuten, neben den üblichen Lösungsmittel, Trägerstoffen und/oder Formulierungshilfsstoffen enthalten.

Die erfindungsgemäßen Verbindungen können in zwei verschiedenen geometrischen Strukturen als N-Alkyl-2,6-cis-dimethylmorpholinio-carbonsäureamid-Salz bzw. N-Alkyl-2,6-trans-dimethylmorpholinio-carbonsäureamid-Salz oder als Gemische dieser beiden Isomeren vorliegen.

Für die fungizide Anwendung kann man sowohl das Isomerengemisch, wie es bei der Synthese anfällt als auch die beiden getrennten Isomeren verwenden.

Oberraschenderweise wurde gefunde, daß die erfindungsgemäßen N-Alkyl-2,6-dimethylmorpholinio-carbonsäureamid-Salze der allgemeinen Formel I eine starke fungizide Wirksamkeit und ein breites Wirkungsspektrum besitzen und sich insbesondere zur Bekämpfung von phytopathogenen Pilzen an Kulturpflanzen und pflanzlichen Vorratsgütern eignen. Die Wirkstoffe zeigen eine gute Pflanzenverträglichkeit bei den zur Bekämpfung von Pflanzenkrankheiten erforderlichen Aufwandmengen. Zusätzlich können die erfindungsgemäßen Verbindungen mit ihren wachstumsregulierenden Eigenschaften Kulturpflanzen in gewünschter Weise positiv beeinflussen.

Weiterhin wurde gefunden, daß die N-Alkyl-2,6-dimethylmorpholiniocarbonsäureamid-Salze der allgemeinen Formel I erhalten werden, indem man ein N-Alkyl-2,6-dimethylmorpholin der Formel II,

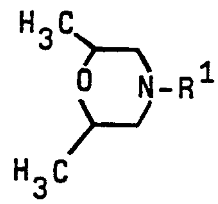

(II),

worin $R^1$ die in der allgemeinen Formel I angegebene Bedeutung besitzt, mit einer Verbindung der Formel III,

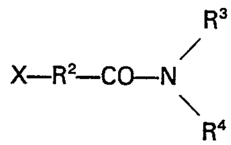

(III)

worin $R^2$, $R^3$ und $R^4$ die in der allgemeinen Formel I angegebenen Bedeutung besitzen und X für Halogen steht, umsetzt oder alternativ ein 2,6-Dimethylmorpholino-carbonsäureamid der Formel IV,

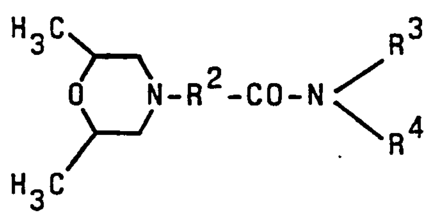

(IV),

worin $R^2$, $R^3$ und $R^4$ die in der allgemeinen Formel I angegebene Bedeutung besitzen, mit einer Verbindung der Formel V,

$$R^1—X \qquad (V)$$

worin $R^1$ die in der allgemeinen Formel I angegebene Bedeutung besitzt und X für Halogen steht, umsetzt.

N-Alkyl-2,6-Dimethylmorpholine der Formel II sind z.B. n-Octyl-, n-Dodecyl-, n-Tridecyl-, iso-Tridecyl-, Pentadecyl- oder n-Didecyl-2,6-dimethylmorpholin.

Halogencarbonsäureamide der Formel III sind beispielsweise Chloracetamid, Chloressigsäure-N-$C_1$—$C_{20}$-alkylamid, -N-2-cyanoethylamid, -N,N-diethylamid, -N,N-bis-(2-cyanoethyl)-amid, -N-cyclohexylamid, -anilid, -4-chloranilid, -3,5-dichloranilid, 3,5-dichlor-4-methoxyanilid, -1H-1,2,4-triazol-1-yl-amid, 2-Brompropionsäure-3,5-dichloranilid, -2,6-dibrom-4-nitroanilid, -N-methyl-2,6-dichlor-4-cyanoanilid oder -2,6-dibrom-4-thiocyanato-anilid.

2,6-Dimethylmorpholino-carbonsäureamide der Formel IV sind z.B. 2,6-dimethylmorpholino-essigsäureamid, -essigsäure-N-$C_1$—$C_{20}$-alkylamid, -essigsäure-bis(2-cyanoethyl)-amid, -essigsäure-N-cyclohexylamid, -essigsäure-anilid, -essigsäure-4-chloranilid, -essigsäure-3,5-dichloranilid, -essigsäure-3,5-dichlor-4-methoxyanilid, -2-propionsäure-3,5-dichloranilid, -2-propionsäure-2,6-dibrom-4-nitroanilid, -2-propionsäure-2,6-dibrom-4-thiocyanato oder -2-propionsäure-N-methyl-2,6-dichlor-4-cyanoanilid.

Alkylhalogenide der Formel V sind beispielsweise n-Octylchlorid, n-Dodecylchlorid, n-Tridecylchlorid,

iso-Tridecylchlorid, 1,5,9-Trimethyldecylchlorid, Pentadecylbromid oder Didecylbromid.

Die Umsetzung zu den erfindungsgemäßen Verbindungen der Formel I werden gegebenenfalls in Gegenwart eines Lösungs- oder Verdünnungsmittels bei einer Temperatur im Bereich zwischen 10 und 180°C, vorzugsweise zwischen 30 und 150°C, durchgeführt. Die Ausgangsstoffe der formel II bzw. der Formel IV werden in stöchiometrischen Mengen mit einer Verbindung der Formel III bzw. der Formel V oder bevorzugt mit einem Überschuß von 10 bis 100% an einer Verbindung der Formel III bzw. der Formel V über die stöchiometrische Menge hinaus, bezogen auf die Ausgangsstoffe der Formel II bzw. der Formel IV, umgesetzt.

Als bevorzugte Lösungs- oder Verdünnungsmittel können beispielsweise aliphatische oder aromatische, gegebenenfalls halogenierte Kohlenwasserstoffe, wie n-Pentan, Cyclohexan, Benzen, Toluen, Chlorbenzen, Chloroform oder Methylenchlorid; aliphatische Ketone, wie Aceton, Methylethylketon oder Cyclohexanon; Ether, wie Diethylether, Tetrahydrofuran oder Dioxan; Alkohole, wie Methanol, Ethanol, Propanole, Butanole oder Hexanole; Nitrile, wie Acetonitril; Ester, wie Essigsäuremethylester; Amide, wie Dimethylformamid, Dimethylacetamd oder N-Methyl-pyrrolidon; Dimethylsulfoxid oder Wasser oder Gemische dieser Lösungsmittel verwendet werden.

Die isolierung der erfindungsgemäßen Verbindungen der allgemeinen Formel I aus den Reaktionsmischungen ist nicht unbedingt erforderlich, da sie auch ohne weitere Reinigungsoperation zur Herstellung fungizider Zubereitungen einsetzbar sind.

Die erfindungsgemäßen Wirkstoffe der allgemeinen Formel I besitzen eine starke Wirksamkeit gegen Mikroorganismen und können dementsprechend zur Bekämpfung von pilzlichen Schaderregern in der Landwirtschaft und im Gartenbau Verwendung finden. Mit den Wirkstoffen können an Pflanzen oder Pflanzenteilen auftretende unerwünschte Pilze bekämpft werden. Die Wirkstoffe der allgemeinen Formel I eignen sich ferner als Beizmittel zur Behandlung von Saatgut und Pflanzenstecklingen zum Schutz vor Pilzinfektionen und können gegen im Erdboden auftretenden phytopathogene Pilze eingesetzt werden. Zusätzlich beeinflussen die wirkstoffe bei ihrer Anwendung die Wachstumsprozesse von Kulturpflanzen in positiver Weise.

Die erfindungsgemäßen Wirkstoffe eignen sich besonders zur Verhütung und Heilung von Pflanzenkrankheiten, die durch Pilze verursacht werden, wie z.B. Erysiphe graminis (Getreidemehltau), Erysiphe cichoracearum (Gurkenmehltau), Erysiphe polygoni (Bohnenmehltau); Podosphaera leucotricha (Apfelmehltau), Sphaerotheca pannosa (Rosenmehltau), Uncinula necator (Rebenmehltau); Rostkrankheiten, wie solche der Gattungen Puccinia, Uromyces oder Hemileia, insbesondere Puccinia graminis (Getreideschwarzrost), Puccinia coronata (Haferkronenrost), Puccinis sorghi (Maisrost), Puccinia recondita (Getreidebraunrost), Uromyces fabae (Buschbohnenrost), Hemileia vastratrix (Kaffeerost); Botrytis cinerea an Reben und Erdbeeren; Monilia fructigena an Äpfeln; Plasmopara viticola an Reben; Mycosphaerella musicola an Bananen; Corticum salmonicolor an Hevea; Ganoderma pseudoferrum an Hevea; Exobasidium vexans an Tee; Phytophthora infestans an Kartoffeln und Tomaten; Alternaria solani an Tomaten. Ferner sind verschiedene dieser Wirkstoffe auch unterschiedlich wirksam gegen phytopathogene Pilze, wie z.B. Ustilago avenae (Flugbrand), Ophiobolus graminis (Getreidefußkrankheiten), Septoria nodorum (Getreideblatt- und Spelzenbräune), Venturia inaequalis (Apfelschorf) sowie weitere pilzliche Krankheitserreger, wie Rhizoctonia, Tilletia, Helminthosporium, Peronospora, Pythium, Alternaria, Mucor, Sclerotinia, Fusarium, Pseudocercosporella und Cladosporium.

Besonders interessant sind die erfindungsgemäßen Wirkstoffe für die Bekämpfung einer Vielzahl von Pilzkrankheiten an verschiedenen Kulturpflanzen oder ihren Samen, insbesondere Weizen, Roggen, Gerste, Hafer, Reis, Mais, Baumwolle, Soja, Kaffee, Bananen, Zuckerrohr, Obst, Zierpflanzen im Gartenbau, Gemüse, wie Gurken, Bohnen oder Kürbisgewächse.

Zusätzlich können die erfindungsgemäßen Wirkstoffe mit ihrem pflanzenwachtumsregulierenden Eigenschaften die Entwicklung von Kulturpflanzen in gewünschter Weise positiv beeinflussen. Die Wirkungen der Verbindungen sind im wesentlichen von dem Zeitpunkt der Anwendung, bezogen auf das Entwicklungsstadium des Samens oder der Pflanzen, von den auf die Pflanzen oder ihre Umgebung ausgebrachten Wirkstoffmengen und von der Art der Applikation abhängig.

Weiterhin erbringen die Wirkstoffe auch eine gute Wirksamkeit gegen holzverfärbende und holzzerstörende Pilze, wie z.B. Pullaria pullulans, Aspergillus niger, Polystictus versicolor oder Chaetomium globosum.

Ferner zeigen die erdingungsgemäßen Wirkstoffe der allgemeinen formel I eine gute Aktivität gegen Schimmelpilze, wie z.B. Penicillium-, Fusarium- oder Aspergillus-Arten, die einen Verderb von hochfeuchtigkeitschaltigen landwirtschaftlichen Produkten oder Verarbeitungsprodukten landwirtschaftlicher Erzeugnisse während der Lagerung oder Zwischenlagerung verursachen. Derartig zu behandelnde Produkte umfassen z.B. Äpfel, Apfelsinen, Mandarinen, Zitronen, Pampelmusen, Erdnüsse, Getreide und Getreideprodukte oder Hülsenfrüchte und -schrot.

Die erfindungsgemäßen Wirkstoffe sind neben ihrem breiten fungiziden Wirkungsspektrum auch unterschiedlich gegen phytopathogene Bakterien, wie beispielsweise Xanthomonas- oder Erwinia-Arten, wirksam.

Einige der Wirkstoffe zeigen auch eine Wirkung gegen humanpathogene Pilze, wie z.B. Trichophyten- und Candida-Arten.

Ein Teil der Wirkstoffe der allgemeinen Formel I zeichnet sich neben der protektiven Wirkung durch

eine systematische Wirkungsentfaltung aus. So werden sie sowohl über die Wurzel als auch über die Blätter aufgenommen und im Pflanzengewebe transportiert oder über das Saatgut den oberirdischen Teilen der Pflanze zugeführt.

Die erfindungsgemäßen Wirkstoffe sind ferner geeignet zur Bekämpfung resistenter Stämme pilzlicher Schaderreger, die gegen bekannte fungizide Wirkstoffe, wie z.B. Wirkstoffe aus der Gruppe der Dicarboximid-Fungizide, wie beispielsweise 5-Methyl-5-vinyl-3-(3,5-dichlorphenyl)-2,4-dioxo-1,3-oxazolidin (Vinclozolin) oder 5-Methyl-5-methoxymethyl-3-(3,5-dichlorphenyl)-1,3-oxazolidin-2,4-dion (Myclozolin); Wirkstoffe aus der Gruppe der Benzimidazol- oder Thiophanat-Fungizide, wie beispielsweise 1-(n-Butylcarbamoyl)-benzimidazol-2-yl-carbaminsäuremethylester (Benomyl), Benzimidazol-2-yl-carbaminsäuremethylester (Carbendazim) oder 1,2-Bis-(3-ethoxycarbonyl-2-thioureido)-benzen (Thiophanat); Wirkstoffe aus der Gruppe der Azol-Fungizide, wie beispielsweise 1-(4-Chlorphenoxy-3,3-dimethyl-1-(1H-1,2,4-triazol-1-yl)-butan-2-on (Triadimefon) oder 1-[2′-(2″,4′-Dichlorphenyl)-2′-(propenyloxy)-ethyl]-1,3-imidazol (Imazalil); Wirkstoffe aus der Gruppe der aromatischen Kohlenwasserstoffe enthaltenden Fungizide, wie beispielsweise 2,5-Dichlor-1,4-dimethoxy-benzen (Chloroneb) oder 2,6-Dichlor-4-nitro-anilin (Dichloran); Wirkstoffe aus der Gruppe der Acylalanin-Fungizide, wie beispielsweise DL-N-(2,6-dimethylphenyl)-N-(2′-methoxyacetyl)alanin-methylester (Metalaxyl) oder DL-N-(2,6-dimethylphenyl)-N-(2-furoyl)-alanin-methylester (Furalaxyl) oder Wirkstoffe aus der Gruppe der Pyrimidin-Fungizide, wie beispielsweise 5-Butyl-2-dimethylamino-4-hydroxy-6-methyl-pyrimidin (Dimethirimol) oder 2-Chlorphenyl-4-chlorphenyl-pyrimidin-5-yl-methanol (Fenarimol), Resistenzerscheinungen zeigen.

Die in der allgemeinen formel I der erfindungsgemäßen Wirkstoffe aufgeführten Anionen $X^-$ sind für fungizide Wirkung nicht ausschlaggebend.

Die erfindungsgemäßen Wirkstoffe können in die üblichen Formulierungen überführt werden, wie Lösungen, Emulsionen, Spritzpulver, Suspensionen, Pulver, Stäubemittel, Schäume, Pasten, lösliche Pulver, Granulate, mit Wirkstoff imprägnierte Natur- und synthetische Stoffe, Feinstverkapselungen in polymeren Stoffen und in Hüllmassen für Saatgut sowie ULV-Kalt- und Warmnebel-Formulierungen.

Die Herstellung dieser Formulierungen kann in bekannter Weise erfolgen, z.B. durch Vermischen oder vermahlen der erfindungsgemäßen Wirkstoffe der allgemeinen Formel I mit Lösungsmitteln und/oder Trägerstoffen, gegebenenfalls unter Verwendung von oberflächenaktiven Mitteln, wie Emulgiermitteln und/oder Dispergiermitteln.

Als flüssige Lösungsmittel können Aromaten, wie Toluen, Xylen oder Alkylnaphthalene; chlorierte Aromaten oder chlorierte aliphatische Kohlenwasserstoffe, wie Chlorbenzene, Chlorethylene oder Methylenchlorid; aliphatische Kohlenwasserstoffe, wie Cyclohexan oder Paraffine, z.B. Erdölfraktionen; Alkohole, wie Butanole oder Glykole sowie deren Ester und Ether; Ketone, wie Aceton, Methylethylketon, Methylisobutylketon oder Cyclohexanon; stark polare Lösungsmittel wie Wasser, Dimethylformamid oder Dimethylsulfoxid verwendet werden. Ebenso können verflüssigte Lösungsmittel, die unter Normalbedingungen gasförmig sind, wie z.B. Aerosol-Treibgase, wie Halogenkohlenwasserstoffe, Propan, Butan, Stickstoff und Kohlendioxid eingesetzt werden.

Als feste Trägerstoffe kommen z.B. natürliche Gesteinsmehle, wie Kaoline, Tonerden, Talkum, Kreide, Quarz, Montmorillonit oder Diatomeenerde und synthetische Gesteinsmehle, wie hochdisperse Kieselsäure, Aluminiumoxid und Silikate in Frage. Als feste Trägerstoffe für Granulate können z.B. gebrochene und fraktionierte natürliche Gesteinsmehle, wie Calcit, Marmor, Bims, Dolomit sowie synthetische Granulate aus anorganischen oder organischen Mehlen sowie Granulate aus organischen Materialien, wie Sägemehl, Cellulosepulver, Baumrinden- und Nußschalenmehl, verwendet werden.

Als Emulgiermittel können z.B. nichtionogene und anionische Emulgatoren, wie Alkali-, Erdalkali- oder Ammoniumsalze von Ligninsulfonsäure, Naphthalensulfonsäuren, Phenolsulfonsäuren, Alkylarylsulfonate, Alkylsufate, Alkylsulfonate, Alkali- und Erdalkalisalze der Dibutylnaphthalensulfonsäure, Laurylethersulfat, Fettalkoholsulfate, fettsaure Alkali- und Erdalkalisalze, Salze sulfatierter Hexadecanole, Heptdecanole oder Octadecanole, Salze von sulfonierten Fettalkoholglykolethern, Kondensationsprodukte von sulfonierten Naphthalen und Naphthalenderivaten mit Formaldehyd, Kondensationsprodukte des Naphthalens bzw. der Naphthalensulfonsäuren mit Phenol und Formaldehyd, Polyoxyethylen-octylphenolether, Alkylphenolpolyglykolether, Tributylphenylpolyglykolether, Alkylarylpolyetheralkohole, iso-Tridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal und Sorbitester, eingesetzt werden.

Als Dispergiermittel kommen beispielsweise Lignin-Sulfitablaugen und Methylcellulose in Betracht.

Es können in der Formulierungen Haftmittel, wie Carboxymethylcellulose, natürliche und synthetische latexförmige Polymere, wie Gummiarabicum, Polyvinylalkohol und Polyvinylacetat, verwendet werden.

Als weitere Zusätze können Farbstoffe und Spurennährstoffe in den Formulierungen der Wirkstoffe enthalten sein.

Die Formulierungen enthalten im allgemeinen zwischen 0,1 und 95 Masseprozent Wirkstoff, vorzugsweise zwischen 0,5 und 90 Masseprozent.

Das erfindungsgemäße Verfahren zur Bekämpfung von Pilzen ist dadurch gekennzeichnet, daß man fungizide Mittel mit den erfindungsgemäßen Wirkstoffen der allgemeinen Formel I auf Pilze oder die vor Pilzbefall zu schützenden Gegenstände in wirksamer Menge einwirken läßt.

Die erfindungsgemäßen Wirkstoffe können in den Formulierungen oder in den verschiedenen Anwendungsformen mit anderen bekannten Wirkstoffen, wie Fungiziden, Bakteriziden, Insektiziden,

Akariziden, Nematiziden, Herbiziden, Wachstrumsregulatoren, Pflanzennährstoffen und Bodenstrukturverbesserungsmitteln vermischt und ausgebracht werden. In vielen Fällen erhält man bei der Mischung mit Fungiziden eine Verbreiterung des fungiziden Wirkungsspektrums. Bei einer Anzahl von Mischungen der erfindungsgemäßen Wirkstoffe mit bekannten Fungiziden treten auch synergistische Effekte auf, wobei die fungizide Wirksamkeit des Kombinationsproduktes größer ist als die der addierten Wirksamkeit der Einzelkomponenten.

Fungizide, die mit den erfindungsgemäßen Wirkstoffen kombiniert werden können, ohne dabei die Kombinationsmöglichkeiten einzuschränken sind beispielsweise:

Schwefel,
Dithiocarbamate und deren Derivate, wie Ferridimethyldithiocarbamat,
Zinkdimethyldithiocarbamat, Manganethylenbisdithiocarbamat, Mangan-Zink-ethylendiamin-bis-dithiocarbamat und Zinkethylenbisdithiocarbamat,
Tetramethylthiuramsulfide,
Ammoniak-Komplex von Zink-(N,N-ethylen-bis-dithiocarbamat) und N,N'-Polyethylen-bis-(thiocarbamoyl)-disulfid,
Ammoniak-Komplex von Zink-(N,N'-propylen-bis-dithiocarbamat) und N,N'-Propylen-bis-(thiocarbamoyl)-disulfid,
N-Trichlormethylthio-tetrahydrophthalimid,
N-Trichlormethylthio-phthalimid,
N-(1,1,2,2-Tetrachlorethylthio)-tetrahydrophthalimid,
4,6-Dinitro-2-(1-(methylheptyl)-phenylcrotonat,
4,6-Dinitro-2-sek.-butylphenyl-3,3-dimethylacrylat,
4,6-dinitro-2-sek.-butylphenyl-isopropylcarbonat,
1-(n-Butylcarbamoyl)-benzimidazol-2-yl-carbaminsäuremethylester,
Benzimidazol-2-yl-carbaminsäuremethylester,
2-(Fur-2-yl)-benzimidazol,
2-(Thiazol-4-yl)-benzimidazol,
1,2-Bis-(3-methoxycarbonyl-2-thioureido)-benzen,
1,2-Bis-(3-ethoxycarbonyl-2-thioureido)-benzen,
2,3-Dihydro-6-methyl-5-phenylcarbamoyl-1,4-oxathiin,
2,3-Dihydro-6-methyl-5-phenylcarbamoyl-1,4-oxathiin-4,4-dioxid,
Tetrachlor-isophthalsäuredinitril,
2,3-Dichlor-1,4-naphthochinon,
2,3-Dicyano-1,4-dithioanthrachinon,
N-Tridecyl-2,6-dimethylmorpholin bzw. dessen Salze,
N-$C_{10}$—$C_{14}$-Alkyl-2,5- und/oder 2,6-dimethylmorpholin,
N-Cyclododecyl-2,6-dimethylmorpholin bzw. dessen Salze,
N-[3-p-tert.-Butylphenyl)-2-methyl-propyl]-2,6-cis-dimethylmorpholin bzw. dessen Salze,
N,N'-Bis-(1-Formamido-2,2,2-trichlorethyl)-piperazin,
N-(1-Formamido-2,2,2-trichlorethyl)-3,4-dichloranilin,
N-(1-Formamido-2,2,2-trichlorethyl)-morpholin,
5-Butyl-2-ethylamino-4-hydroxy-6-methyl-pyrimidin,
5-Butyl-2-dimethylamino-4-hydroxy-6-methyl-pyrimidin
2,4-Dichlorphenyl-phenyl-pyrimidin-5-yl-methanol,
2-Chlorphenyl-4-chlorphenyl-pyrimidin-5-yl-methanol,
Bis-(4-chlorphenyl)-pyridin-3-yl-methanol,
5-Methyl-5-vinyl-3-(3,5-dichlorphenyl)-1,3-oxazolidin-2,4-dion,
5-Methyl-5-methoxymethyl-3-(3,5-dichlorphenyl)-1,3-oxazolidin-2,4-dion,
3-(3,5-Dichlorphenyl)-N-isopropyl-2,4-dioxo-imidazolidin-1-carboxamid,
N-(3,5-Dichlorphenyl)-1,2-dimethylcyclopropan-1,2-dicarboximid,
1-(4-Chlorphenoxy)-3,3-dimethyl-1-(1,2,4-triazol-1-yl)-butan-2-on,
1-(4-Chlorphenoxy)-3,3-dimethyl-1-(1,2,4-triazol-1-yl)-butan-2-ol,
1-[2-(2,4-Dichlorphenyl)-2-(propenyloxy)-ethyl]-imidazol,
1-[N-Propyl-N-(2,4,6-trichlorphenoxy)-ethyl-carbamoyl]-imidazol,
1-[2-(2,4-Diclorphenyl)-4-ethyl-1,3-dioxolan-2-yl-methyl]-1H-1,2,4-triazol,
1-[2-(2,4-Diclorphenyl)-4-n-propyl-1,3-dioxolan-2-yl-methyl]-1H-1,2,4-triazol,
1-(2,4-Dichlorphenyl)-2-(1,2,4-triazol-1-yl)-4,4-dimethyl-pentan-3-on,
2,5-Dichlor-1,4-dimethoxy-benzen,
2,6-Dichlor-4-nitroanilin,
Diphenyl,
2-Methyl-benzoesäure-anilid,
2-Jod-benzoesäure-anilid,
2,5-Dimethyl-furan-3-carbonsäureanilid,
2,4,5-Trimethyl-furan-3-carbonsäureanilid,
N-Cyclohexyl-N-methoxy-2,5-dimethyl-furan-3-carbonsäureamid,

3-[3-(3,5-Dimethyl-2-oxycyclohexyl)-2-hydroxyethyl]-glutarimid,
N-Dichlorfluormethylthio-N',N'-dimethyl-N-phenyl-schwefelsäurediamid,
DL-N-(2,6-Dimethylphenyl)-N-(2'-methoxyacetyl)-alanin-methylester,
DL-N-(2,6-Dimethylphenyl)-N-(2-furoyl)-alanin-methylester,
N-(2,6-Dimethylphenyl)-N-chloracetyl-DL-2-aminobutylrolacton,
2,4-Dichlor-6-(2-chloranilino)-s-triazin,
O,O-Diethyl-phthalimido-phosphonothioat,
5-Amino-1-(bis-(dimethylamino)-phosphinyl)-3-phenyl-1,2,4-triazol,
O,O-Diethyl-S-benzyl-thiophosphorsäureester,
2-Thio-1,3-dithio-(4,5-b)-chinoxalin),
4-(2-Chlorphenylhydrazono)-3-methyl-5-isoxalon,
Pyridin-2-thiol-1-oxid,
8-Hydroxychinolin bzw. dessen Salze,
4-Dimethylaminophenyl-diazo-sulfonsäure-Natriumsalz,
5-Nitro-isophthalsäure-di-isopropylester,
2-Cyan-N-(ethylaminocarbonyl)-2-(methoxyimino)-acetamid,
2-Heptadecyl-2-imidazolin-acetat,
Dodecyl-guanidinacetat.

Die Wirkstoffe können als solche, in Form ihrer Formulierungen oder den daraus durch weiteres Verdünnen bereiteten Anwendungsformen in üblicher Weise, z.B. durch Gießen, Tauchen, Spritzen, Sprühen, Vernebeln, Injizieren, Verschlämmen, Verstreichen, Stäuben, Streuen, Trockenbeizen, Feuchtbeizen, Naßbeizen, Schlämmbeizen oder Inkrustieren, angewendet werden.

Bei der Behandlung von Pflanzenteilen können die Wirkstoffkonzentrationen in den Anwendungsformen in einem größeren Bereich variiert werden. Sie liegen im allgemeinen zwischen 0,0001 und 1 Masseprozent, vorzugsweise zwischen 0,5 und 0,001 Masseprozent. Die Wirkstoffaufwandmengen sind vom spezifischen Anwendungszweck abhängig und liegen im allgemeinen zwischen 0,1 und 3 kg Wirkstoff pro Hektar.

Bei der Saatgutbehandlung werden im allgemeinen wirkstoffmengen von 0,001 bis 50 g oder mehr je Kilogramm Saatgut, vorzugsweise 0,01 bis 10 g, benötigt.

Zur Konservierung oder Nacherntebehandlung von landwirtschaftlichen Produkten oder Verarbeitungsprodukten landwirtschaftlicher Erzeugnisse betragen die benötigten Wirkstoffmengen 0,01 bis 100 g je Kilogramm Behandlungsgut, vorzugsweise 0,1 bis 50 g.

Bei der Behandlung des Bodens sind Wirkstoffkonzentrationen von 0,0001 bis 0,1 Masseprozent, vorzugsweise von 0,001 bis 0,05 Masseprozent, am Wirkungsort erforderlich.

Die nachfolgenden Beispiele sollen die Erfindung weiter erläutern, ohne sie jedoch einzuschränken und die Wirkung der erfindungsgemäßen Verbindungen der allgemeinen Formel I belegen.

## Ausführungsbeispiele

Die nachfolgenden Beispiele erläutern die Herstellung der erfindungsgemäßen Verbindungen und ihrer Vorprodukte.

Die Ausgangsverbindungen zur Herstellung der erfindungsgemäßen Verbindungen sind an sich bekannt oder können nach an sich bekannten Verfahren hergestellt werden, wie aus den nachstehend aufgeführten Synthesevorschriften hervorgeht:

Vorschrift zur Synthese der Halogencarbonsäureamide der Formel III:

### Herstellung von Chloressigsäure-N,N-(2-cyanoethyl)-amid (Vorprodukt für die Verbindung Nr. 32)

Zu einer Lösung von 12,3 g Bis-(2-cyanoethyl)-amin in 100 ml getrocknetem Dioxan werden 14 ml Triethylamin gegeben. Anschließend werden 12,5 g Chloracetylchlorid (8,8 ml) in 10 ml getrocknetem Dioxan innerhalb 1 Stunde zugetropft. Die Temperatur wird dabei unterhalb von 30°C gehalten. Nach 2 Stunden Rühren bei Raumtemperatur wird das Reaktionsgemisch auf 200 ml Eiswasser gegossen. Man extrahiert das Produkt mit Essigsäureethylester, wäscht die organische Phase mit Wasser, trocknet über Natriumsulfat und engt im Vakuum ein. Das Rohprodukt wird aus Aceton/n-Hexan umkristallisiert. Ausbeute 14 g. Schmp. 68—68,5°C.

Vorschrift für die Synthese der 2,6-Dimethylmorpholino-carbonsäureamide der Formel IV:

### Herstellung von 2-(2,6-cis-dimethylmorpholino)-propionsäure-3,5-dichloranilid (Vorprodukt für die Verbindung Nr. 80)

22,6 g 2,6-cis-Dimethylmorpholin werden in 100 ml getrocknetem Dioxan gelöst. Dazu wird eine Lösung von 29,7 g 2-Brompropikonsäure-3,5-dichloranilid in 20 ml getrocknetem Dioxan getropft. Anschliessend wird noch 3 Stunden zum Rückfluß erwärmt. Das Reaktionsgemisch wird im Vakuum zur Trockene eingeengt. Es wird aus Methanol umkristallisiert. Ausbeute 26 g. Schmp. 158—158,5°C.

Beispiel 1

N-iso-Tridecyl-2,6-dmethylmorpholino-essigsäure-N',N'-bis(2-cyanoethyl)-amid-chlorid

30 g N-iso-Tridecyl-2,6-dimethylmorpholin und 20 g Chloressigsäure-N,N-di-(2-cyanoethyl)-amid werden unter Zugabe ener katalytischen Menge Natriumjodid in 100 ml Acetonitril 24 Stunden zum Rückfluß erwärmt. Man kühlt ab und destilliert das Lösungsmittel im Vakuum ab. Das Produkt wird in wenig Diethylether gelöst und mit n-Hexan bis zur vollständigen Abscheidung versetzt. Nach Einengen im Vakuum erhält man 45 g eines gelbbraunen viskosen Öles (Verbindung Nr. 32).

IR-Spektrum (Film): C = O — Absorption 1660 $cm^{-1}$.

Beispiel 2

2-(N-iso-Tridecyl-2,6-dimethylmorpholinio)-propionsäure-3,5-dichloranilid-bromid

30 g N-iso-Tridecyl-2,6-dimethylmorpholin und 29,8 g 2-Brompropionsäure-3,5-dichloranilid werden in 100 ml n-Butanol unter Zugabe einer katalytischen Menge Natriumjodid 24 Stunden zum Rückfluß erwärmt. Man kühlt ab und destilliert das Lösungsmittel im Vakuum ab. Das zurückbleibende Produkt wird in wenig Diethylether gelöst und bis zur vollständigen Abscheidung mit n-Hexan versetzt. Die ölige Phase wird abgetrennt und im Vakuum von Lösungsmittelresten befreit. Man erhält 54 g eines gelbbraunen hochviskosen Öles (Verbindung Nr. 80).

IR-Spektrum (Film): C = O — Absorption 1690 $cm^{-1}$.

Beispiel 3

N-iso-Tridecyl-2,6-dimethylmorpholinio-essigsäure-3,5-dichloranilid-chlorid

31,7 g 2,6-Dimethylmorpholino-essigsäure-3,4-dichloranilid und 21,9 g iso-Tridecylchlorid (Gemisch verschiedener $C_{11}$—$C_{14}$-Alkylchloride, das 60 bis 70% n-Tridecylchlorid enthält) in 100 ml Dimethylformamid werden 12 Stunden zum Rückfluß erhitzt. Nach dem Abkühlen wird das Lösungsmittel im Vakuum abdestilliert. Das Rohprodukt wird in wenig Diethylether gelöst und bis zur vollständigen Abscheidung mit n-Hexan versetzt. Die ölige Phase wird abgetrennt und im Vakuum von Lösungsmittelresten befreit. Man erhält 48 g eines hellbraunen Harzes (Verbindung Nr. 75).

IR-Spektrum (Film): C = O — Absorption 1690 $cm^{-1}$.

In entsprechender Weise werden die nachfolgend aufgeführten Verbindungen der allgemeinen Formel I hergestellt, die in der Regel gelbe bis braune viskose Öle oder Harze darstellen, in polaren Lösungsmitteln, wie z.B. Alkohole, Aceton, Dimethylformamid und Dimethylsulfoxid, gut löslich sind und durch IR-Spektren charakterisiert werden.

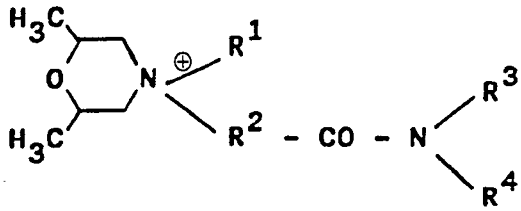

X$^{\ominus}$ (I)

| Nr. | $R^1$ | $R^2$ | $R^3$ | $R^4$ | X |
|---|---|---|---|---|---|
| 1 | n-hexyl | $CH_2$ | H | H | Cl |
| 2 | n-octyl | $CH_2$ | H | H | Cl |
| 3 | n-decyl | $CH_2$ | H | H | Cl |
| 4 | n-dodecyl | $CH_2$ | H | H | Cl |
| 5 | n-tridecyl | $CH_2$ | H | H | Cl |
| 6 | 1,5,9-trimethyldecyl | $CH_2$ | H | H | Cl |
| 7 | iso-tridecyl | $CH_2$ | H | H | Cl |
| 8 | iso-tridecyl | $CH_2$ | H | methyl | Cl |
| 9 | iso-tridecyl | $CH_2$ | H | ethyl | Cl |
| 10 | iso-tridecyl | $CH_2$ | H | sek.-butyl | Cl |
| 11 | iso-tridecyl | $CH_2$ | H | 2-ethylhexyl | Cl |
| 12 | iso-tridecyl | $CH_2$ | H | n-dodecyl | Cl |
| 13 | iso-tridecyl | $CH_2$ | H | n-octadecyl | Cl |
| 14 | iso-tridecyl | $CH_2$ | H | 3-hydroxypropyl | Cl |
| 15 | iso-tridecyl | $CH_2$ | H | 3-ethoxypropyl | Cl |
| 16 | iso-tridecyl | $CH_2$ | H | 2-cyanoethyl | Cl |
| 17 | iso-tridecyl | $CH_2$ | H | 2-diethylaminoethyl | Cl |
| 18 | iso-tridecyl | $CH(CH_3)$ | H | H | Br |
| 19 | iso-tridecyl | $CH(CH_3)$ | H | n-dodecyl | Br |
| 20 | iso-tridecyl | $CH(C_2H_5)$ | H | n-butyl | Br |
| 21 | iso-tridecyl | $C(CH_3)_2$ | H | n-butyl | Br |

| Nr. | $R^1$ | $R^2$ | $R^3$ | $R^4$ | X |
|---|---|---|---|---|---|
| 22 | iso-tridecyl | $CH(C_5H_{11})$ | H | n-butyl | Br |
| 23 | iso-tridecyl | $(CH_2)_3$ | H | n-butyl | Cl |
| 24 | iso-tridecyl | $(CH_2)_6$ | H | n-butyl | Br |
| 25 | $n-C_{15}H_{31}$ | $CH_2$ | H | n-butyl | Cl |
| 26 | $n-C_{20}H_{41}$ | $CH_2$ | H | n-butyl | Cl |
| 27 | iso-tridecyl | $CH_2$ | ethyl | ethyl | Cl |
| 28 | iso-tridecyl | $CH_2$ | n-octyl | n-octyl | Cl |
| 29 | iso-tridecyl | $CH(CH_3)$ | n-butyl | n-butyl | Br |
| 30 | iso-tridecyl | $CH_2$ | 2-hydroxy-ethyl | 2-hydroxyethyl | Cl |
| 31 | n-octyl | $CH_2$ | 2-cyano-ethyl | 2-cyanoethyl | Cl |
| 32 | iso-tridecyl | $CH_2$ | 2-cyano-ethyl | 2-cyanoethyl | Cl |
| 33 | iso-tridecyl | $CH_2$ | methyl | 3-cyanopropyl | Cl |
| 34 | iso-tridecyl | $CH_2$ | H | allyl | Cl |
| 35 | iso-tridecyl | $CH(CH_3)$ | allyl | allyl | Br |
| 36 | iso-tridecyl | $CH_2$ | methyl | crotyl | Cl |
| 37 | iso-tridecyl | $CH_2$ | methyl | 4-chlorbut-2-en-1-yl | Cl |
| 38 | iso-tridecyl | $CH_2$ | methyl | propargyl | Cl |
| 39 | iso-tridecyl | $CH_2$ | H | cyclopropyl | Cl |
| 40 | iso-tridecyl | $CH(CH_3)$ | H | cyclopentyl | Br |
| 41 | iso-tridecyl | $CH(CH_3)$ | H | cyclohexyl | Br |
| 42 | iso-tridecyl | $CH_2$ | H | cycloheptyl | Cl |
| 43 | iso-tridecyl | $CH_2$ | ethyl | cyclohexyl | Cl |
| 44 | iso-tridecyl | $CH_2$ | H | phenyl | Cl |
| 45 | iso-tridecyl | $CH_2$ | methyl | phenyl | Cl |
| 46 | iso-tridecyl | $CH_2$ | n-propyl | phenyl | Cl |
| 47 | iso-tridecyl | $CH_2$ | allyl | phenyl | Cl |
| 48 | iso-tridecyl | $CH_2$ | propargyl | phenyl | Cl |
| 49 | iso-tridecyl | $CH_2$ | benzyl | phenyl | Cl |
| 50 | iso-tridecyl | $CH(CH_3)$ | allyl | phenyl | Br |
| 51 | iso-tridecyl | $CH_2$ | H | naphth-1-yl | Cl |
| 52 | iso-tridecyl | $CH_2$ | H | biphen-2-yl | Cl |
| 53 | iso-tridecyl | $CH_2$ | H | 2-chlorphenyl | Cl |
| 54 | iso-tridecyl | $CH_2$ | H | 3-fluorphenyl | Cl |

| Nr. | $R^1$ | $R^2$ | $R^3$ | $R^4$ | X |
|---|---|---|---|---|---|
| 55 | iso-tridecyl | $CH_2$ | H | 4-chlorphenyl | Cl |
| 56 | iso-tridecyl | $CH(CH_3)$ | methyl | 3-bromphenyl | Br |
| 57 | iso-tridecyl | $CH_2$ | H | 4-ethylphenyl | Cl |
| 58 | iso-tridecyl | $CH(CH_3)$ | methyl | 4-n-butoxyphenyl | Br |
| 59 | iso-tridecyl | $CH(CH_3)$ | methyl | 4-formylphenyl | Br |
| 60 | iso-tridecyl | $CH(CH_3)$ | methyl | 4-acetophenyl | Br |
| 61 | iso-tridecyl | $CH_2$ | H | 3-nitrophenyl | Cl |
| 62 | iso-tridecyl | $CH_2$ | H | 4-cyanophenyl | Cl |
| 63 | iso-tridecyl | $CH_2$ | H | 3-trifluormethyl-phenyl | Cl |
| 64 | iso-tridecyl | $CH_2$ | H | 4-thiocyanatophenyl | Cl |
| 65 | iso-tridecyl | $CH_2$ | H | 4-acetaminophenyl | Cl |
| 66 | iso-tridecyl | $CH_2$ | H | 4-methoxycarbonyl-phenyl | Cl |
| 67 | iso-tridecyl | $CH_2$ | H | 4-N,N-dimethylamido-carbonylphenyl | Cl |
| 68 | iso-tridecyl | $CH_2$ | H | 4-N,N-dimethylureido-phenyl | Cl |
| 69 | iso-tridecyl | $CH_2$ | H | 4-N,N-dimethylamido-sulfenylphenyl | Cl |
| 70 | iso-tridecyl | $CH_2$ | H | 4-phenylsulfonyl-phenyl | Cl |
| 71 | iso-tridecyl | $CH_2$ | H | 2,3-dichlorphenyl | Cl |
| 72 | iso-tridecyl | $CH_2$ | H | 2,4-dichlorphenyl | Cl |
| 73 | iso-tridecyl | $CH_2$ | H | 2,5-dichlorphenyl | Cl |
| 74 | iso-tridecyl | $CH_2$ | H | 2,6-dibromphenyl | Cl |
| 75 | iso-tridecyl | $CH_2$ | H | 3,4-dichlorphenyl | Cl |
| 76 | n-octyl | $CH_2$ | H | 3,5-dichlorphenyl | Cl |
| 77 | n-octyl | $CH_2$ | methyl | 3,5-dichlorphenyl | Cl |
| 78 | n-octyl | $CH(CH_3)$ | H | 3,5-dichlorphenyl | Br |
| 79 | iso-tridecyl | $CH_2$ | H | 3,5-dichlorphenyl | Cl |
| 80 | iso-tridecyl | $CH(CH_3)$ | H | 3,5-dichlorphenyl | Br |
| 81 | iso-tridecyl | $CH(CH_3)$ | methyl | 3,5-dichlorphenyl | Br |
| 82 | iso-tridecyl | $CH(CH_3)$ | allyl | 3,5-dichlorphenyl | Br |
| 83 | iso-tridecyl | $CH_2$ | H | 3,5-dibromphenyl | Cl |
| 84 | iso-tridecyl | $CH(CH_3)$ | H | 3,5-dibromphenyl | Br |
| 85 | iso-tridecyl | $CH(CH_3)$ | methyl | 3,5-dibromphenyl | Br |
| 86 | iso-tridecyl | $CH_2$ | H | 2,6-diethylphenyl | Cl |

| Nr. | $R^1$ | $R^2$ | $R^3$ | $R^4$ | X |
|---|---|---|---|---|---|
| 87 | iso-tridecyl | $CH_2$ | H | 3,4-dimethylphenyl | Cl |
| 88 | iso-tridecyl | $CH_2$ | H | 3,5-dimethylphenyl | Cl |
| 89 | iso-tridecyl | $CH_2$ | H | 2-nitro-4-trifluor-methylphenyl | Cl |
| 90 | iso-tridecyl | $CH_2$ | H | 2-methyl-4-chlor-phenyl | Cl |
| 91 | iso-tridecyl | $CH(CH_3)$ | H | 2-methyl-4-chlor-phenyl | Br |
| 92 | iso-tridecyl | $CH_2$ | H | 2-cyano-4-nitrophenyl | Cl |
| 93 | iso-tridecyl | $CH_2$ | H | 3,5-dinitrophenyl | Cl |
| 94 | iso-tridecyl | $CH_2$ | H | 2,6-dibrom-4-nitro-phenyl | Cl |
| 95 | iso-tridecyl | $CH_2$ | methyl | 2,6-dibrom-4-nitro-phenyl | Cl |
| 96 | iso-tridecyl | $CH(CH_3)$ | H | 2,6-dibrom-4-nitro-phenyl | Br |
| 97 | iso-tridecyl | $CH(CH_3)$ | methyl | 2,6-dibrom-4-nitro-phenyl | Br |
| 98 | iso-tridecyl | $CH_2$ | H | 2,6-dichlor-4-cyano-phenyl | Cl |
| 99 | iso-tridecyl | $CH_2$ | methyl | 2,6-dichlor-4-cyano-phenyl | Cl |
| 100 | iso-tridecyl | $CH(CH_3)$ | H | 2,6-dichlor-4-cyano-phenyl | Br |
| 101 | iso-tridecyl | $CH(CH_3)$ | methyl | 2,6-dichlor-4-cyano-phenyl | Br |
| 102 | iso-tridecyl | $CH_2$ | H | 3,4,5-trichlorphenyl | Cl |
| 103 | iso-tridecyl | $CH_2$ | methyl | 3,4,5-trichlorphenyl | Cl |
| 104 | iso-tridecyl | $CH_2$ | H | 2,4,6-trichlorphenyl | Cl |
| 105 | iso-tridecyl | $CH_2$ | methyl | 2,4,6-trichlorphenyl | Cl |
| 106 | iso-tridecyl | $CH_2$ | H | 2,6-dibrom-4-thio-cyanato-phenyl | Cl |
| 107 | iso-tridecyl | $CH_2$ | methyl | 2,6-dibrom-4-thio-cyanato-phenyl | Cl |
| 108 | iso-tridecyl | $CH(CH_3)$ | H | 2,6-dibrom-4-thio-cyanato-phenyl | Br |
| 109 | iso-tridecyl | $CH(CH_3)$ | methyl | 2,6-dibrom-4-thio-cyanato-phenyl | Br |
| 110 | iso-tridecyl | $CH_2$ | H | 3,5-dichlor-4-methoxy-phenyl | Cl |

| Nr. | $R^1$ | $R^2$ | $R^3$ | $R^4$ | X |
|---|---|---|---|---|---|
| 111 | iso-tridecyl | $CH_2$ | methyl | 3,5-dichlor-4-methoxyphenyl | Cl |
| 112 | iso-tridecyl | $CH(CH_3)$ | H | 3,5-dichlor-4-methoxyphenyl | Br |
| 113 | iso-tridecyl | $CH(CH_3)$ | methyl | 3,5-dichlor-4-methoxyphenyl | Br |
| 114 | iso-tridecyl | $CH_2$ | H | benzyl | Cl |
| 115 | iso-tridecyl | $CH(CH_3)$ | H | phenethyl | Br |
| 116 | iso-tridecyl | $CH(CH_3)$ | methyl | 4-chlorbenzyl | Br |
| 117 | iso-tridecyl | $CH_2$ | H | 4-methylbenzyl | Cl |
| 118 | iso-tridecyl | $CH_2$ | H | 4-tert.-butylbenzyl | Cl |
| 119 | iso-tridecyl | $CH_2$ | H | 4-methoxybenzyl | Cl |
| 120 | iso-tridecyl | $CH_2$ | H | 3-trifluormethylbenzyl | Cl |
| 121 | iso-tridecyl | $CH_2$ | methyl | 4-cyanobenzyl | Cl |
| 122 | iso-tridecyl | $CH(CH_3)$ | H | 4-nitrobenzyl | Br |
| 123 | iso-tridecyl | $CH_2$ | H | 3,4-dichlorbenzyl | Cl |
| 124 | iso-tridecyl | $CH_2$ | H | 2,3,4-trichlorbenzyl | Cl |
| 125 | iso-tridecyl | $CH_2$ | aziridin-1-yl | | Cl |
| 126 | iso-tridecyl | $CH_2$ | pyrrol-1-yl | | Cl |
| 127 | iso-tridecyl | $CH_2$ | pyrrolidin-1-yl | | Cl |
| 128 | iso-tridecyl | $CH_2$ | pyrazol-1-yl | | Cl |
| 129 | iso-tridecyl | $CH_2$ | 1H-imidazol-1-yl | | Cl |
| 130 | iso-tridecyl | $CH_2$ | 1H-1,2,4-triazol-1-yl | | Cl |
| 131 | iso-tridecyl | $CH_2$ | piperidin-1-yl | | Cl |
| 132 | iso-tridecyl | $CH_2$ | 3,5-dimethylpiperidin-1-yl | | Cl |
| 133 | iso-tridecyl | $CH_2$ | 4-methylpiperazin-1-yl | | Cl |
| 134 | iso-tridecyl | $CH(CH_3)$ | tetrahydro-1,4-oxazin-4-yl | | Br |
| 135 | iso-tridecyl | $CH(CH_3)$ | 2,6-cis/trans-dimethyl-tetrahydro-1,4-oxazin-4-yl | | Br |
| 136 | iso-tridecyl | $CH(CH_3)$ | tetrahydro-1,4-thiazin-4-yl | | Br |
| 137 | iso-tridecyl | $CH(CH_3)$ | 2,6-cis/trans-dimethyl-tetrahydro-1,4- diazin-4-yl | | Br |
| 138 | iso-tridecyl | $CH_2$ | oxazolidin-3-yl | | Cl |
| 139 | iso-tridecyl | $CH_2$ | thiazolidin-3-yl | | Cl |

| Verb. Nr. | IR-Spektren (Film) /$cm^{-1}$/ von erfindungsgemäßen Verbindungen |
|---|---|
| 7 | 3360...3220, 3170...3130, 2960...2900, 2810, 1675, 1620, 1550, 1370, 1135, 1105, 1025 |
| 16 | 3370...3330, 3190, 3020, 2955, 2925, 2865, 1680, 1555, 1455, 1420, 1380, 1240, 1140, 1025 |
| 27 | 3390...3310, 2970...2910, 2875, 1650, 1460, 1380, 1145, 1115, |
| 31 | 3350...3310, 2960...2900, 2850, 2240, 1650, 1450, 1365, 1145, 1100,1060, 1015 |
| 32 | 3390...3330, 2960, 2940, 2880, 2250, 1660, 1470, 1420, 1140, 1035 |
| 44 | 3370...3300, 3265, 3190, 3130, 2980...2900, 2860, 1685, 1600, 1555, 1500, 1455, 1375, 1315, 1145 |
| 55 | 3390...3340, 3220, 3170, 3105, 3015, 2955, 2930, 2870, 1690, 1610, 1550, 1490, 1460, 1395, 1375, 1305, 1245, 1210, 1140, 1090, 1010, 830 |
| 72 | 3390...3370, 3185, 3140, 2970...2920, 2875, 2460, 1700, 1595, 1540, 1490, 1480, 1395, 1300, 1250, 1225, 1155, 1110, 1070, 875, 830 |
| 75 | 3400...3350, 3220, 3150, 3070, 2950, 2920, 2860, 1690, 1585, 1530, 1465, 1370, 1295, 1230, 1125, 1020, 870, 810 |
| 76 | 3380...3340, 3220, 3150, 3090, 3080, 2970...2890, 2860, 1680, 1580, 1540, 1430, 1400, 1360, 1295, 1250, 1195, 1135, 1100, 1010, 830, 790 |
| 79 | 3395...3360, 3225, 3165, 3105, 3025, 2960, 2940, 2870 1695, 1590, 1550, 1445, 1410, 1375, 1310, 1270, 1210, 1145, 1115, 1035, 875, 845, 805 |
| 80 | 3370...3210, 3220, 3165, 3105, 3075, 3045, 2960, 2925, 2870, 2610, 1690, 1585, 1533, 1440, 1405, 1370, 1155, 1110, 1085 |
| 83 | 3390...3350, 3220, 3155, 2960, 2930, 2870, 1695, 1583, 1535, 1435, 1405, 1380, 1300, 1260, 1215, 1140, 1105, 870, 855 |
| 84 | 3360...3210, 3145, 3095, 3070, 2970...2910, 2865, 2620, 1695, 1585, 1525, 1470, 1425, 1405, 1380, 1300, 1255, 1180, 1160, 1115, 1085, 840 |
| 86 | 3360...3240, 3165, 2955, 2920, 2865, 1675, 1580, 1520, 1450, 1370, 1315, 1260, 1220, 1130, 1025, 865 |
| 94 | 3400...3360, 3310...3260, 2960, 2930, 2860, 1695, 1595, 1540, 1520, 1470, 1380, 1350, 1210, 1140, 725 |
| 106 | 2970...2900, 2850, 2160, 1690, 1570, 1470, 1380, 1210, 1140, 725 |
| 104 | 3390...3370, 3150, 3030, 2970, 2940, 2885, 2460, 1705, 1590, 1570, 1540, 1475, 1395, 1230, 1140, 1095, 1060, 875, 835 |
| 114 | 3400...3180, 3065, 3035, 2965, 2935, 2875, 1680, 1610, 1535, 1470, 1460, 1385, 1150, 1120, 1085, 1035, 875 |

Die erfindungsgemäßen Verbindungen der allgemeinen Formel I konnen beispielsweise in Form folgender Zubereitungen zur Anwendung kömmen:

I. Beispiel

Lösungskonzentrate: Man vermischt 80 Gewichtsteile der Verbindung 7 mit 20 Gewichtsteilen N-Methyl-2-pyrrolidon. Es wird ein Lösung erhalten, die zur Anwendung in Form kleinster Tropfen geeignet ist.

II. Beispiel

Emuglierbare Konzentrate: 25 Gewichtsteile der Verbindung 55 werden mit 2,5-Gewichtsteilen epoxylierten Pflanzenöles, 10 Gewichtsteilen eines Alkylarylsulfonat/Fettalkoholpolyglykolether-Gemisches, 5-Gewichtsteilen Dimethylformamid und 57,5 Gewichtsteilen Xylen vermischt. Aus diesem Konzentrat können durch Verdünnen mit Wasser Emulsionen jeder gewünschten Konzentration hergestellt werden.

III. Beispiel

Spritzpulver: 40 Gewichtsteile der Verbindung 86 werden mit 5 Gewichtsteilen des Natrium-Salzes einer Ligninsulfonsäure aus einer Sulfitablauge, 1 Gewichtsteil Natrium-Salz der Di-iso-butylnaphthalensulfonsäure und 54 Gewichtsteilen Kieselsäuregel gut vermischt und in einer entsprechenden Mühle vermahlen. Man erhält ein Spritzpulver, das mit Wasser zu Suspensionen jeder gewünschten Konzentration verdünnt werden kann.

IV. Beispiel

Stäubemittel: 5 Gewichtsteile der Verbindung 80 werden mit 95 Gewichtsteilen feinteiligen Kaolin innig vermischt und vermahlen. Die Stäubemittel können in dieser Form zur Anwendung verstäubt werden.

V. Beispiel

Granulate: 5 Gewichtsteile der Verbindung 86 werden mit 0,25 Gewichtsteilen Epichlorhydrin vermischt und mit 6 Gewichtsteilen Aceton gelöst. Danach werden 3,5 Gewichtsteile Polyethylenglykol und 0,25 Gewichtsteile Cetylpolyglykolether zugesetzt. Die so erhaltene Lösung wird auf Kaolin aufgesprüht. Anschließend wird das Aceton in Vakuum verdampft. Es wird ein Mikrogranulat erhalten, das in dieser Form zur Anwendung kommen kann.

Die nachfolgenden Beispiele sollen die Erfindung weiter erläutern, ohne sie jedoch einzuschränken, und die Wirkung der Verbindungen der allgemeinen Formel I belegen:

## Beispiel A

Mycelwachstums-Test

Die fungizide Wirkung der Mittel gegenüber den Testpilzen wird in herkömmlicher Weise als Hemmung des radialen Mycelwachstums auf Malzagar-Nährboden (2% Malz) in Petrischalen von 9 cm Durchmesser bei einer Inkubationstemperatur von 25°C bestimmt. Die Wirkstoffe werden dazu in Dimethylformamid gelöst, mit Wasser verdünnt und so den flüssigen Agar zugemischt, daß die gewünschte Wirkstoffkonzentration im Nährmedium erreicht wird. Der DMF-Gehalt übersteigt dabei 0,5 Volumenprozent nicht. Nach dem Erkalten werden die Platten beimft. Die Auswertung erfolgt je nach Wachstrumsgeschwindigkeit der Pilze wenn die Kontrollen ohne Wirkstoffzusatz zu 70 bis 90% des Schalendurchmessers durchwachsen sind. Zur Auswertung wird die prozentuale Wachstumshemmung der Wirkstoffe gegenüber den Kontrollen ohne Wirkstoffzusatz berechnet (Tabelle A).

TABELLE A

Wachstumshemmung von Pilzen im Mycelwachstums-Test

| Verbindung Nr. | Wirkstoffkonz. | Wachstumshemmung in Prozent Botrytis cinerea 10 µg/ml | Phytophthora cactorum 50 µg/ml |
|---|---|---|---|
| Oxycarboxin (bekannt) | | 20 | 50 |
| | 7 | 57 | |
| | 16 | 36 | 72 |
| | 27 | 83 | |
| | 31 | 43 | |
| | 32 | 71 | |
| | 41 | 62 | |
| | 44 | 89 | 110 |
| | 55 | 77 | 93 |
| | 72 | 74 | |
| | 75 | 76 | 65 |
| | 79 | 74 | 70 |
| | 80 | 80 | |
| | 83 | 58 | |
| | 84 | 69 | 56 |
| | 86 | 63 | |
| | 94 | 82 | |
| | 114 | 43 | |
| | 129 | 71 | |
| | 130 | 88 | |

Beispiel B

Gerstenmehltau-Test (Erysiphe graminis/Gerste)

In Röhrchem mit Sand werden Gerstenpflanzen der Sorte "Astacus" im Einblattstadium taufeucht mit Wirkstoffzubereitungen besprüht, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach dem Antrocknen des Spritzbelages werden die Pflanzen mit Konidien des Gerstenmehltaus (Erysiphe graminis var. hordei) bestäubt. Anschließend werden die Versuchspflanzen für einen Zeitraum von 2 bis 3 Stunden bei 90 bis 100% relativer Luftfeuchtigkeit in einer Inkubationskabine und danach bei Temperaturen zwischen 20 und 22°C und 75 bis 80% relativer Luftfechtigkeit im Gewächshaus aufgestellt.

Nach 7 Tagen wird der Mehltaubefall der Gerstenpflanzen bestimmt. Die nach STEPHAN (Arch. Phytopath. Pfl.-schutz *14* (1978), 163—175) erhaltenen Boniturwerte werden in den Befallsgrad nach

KRÜGER (Nachr.-Bl. Pflanzenschutz DDR *1981*, 145—147) umgerechnet, Daraus ergibt sich der Wirkungsgrad nach ABBOTT entsprechend

$$\text{WG (in \%)} = \frac{\text{Befallsgrad Kontrolle} - \text{Befallsgrad Variante}}{\text{Befallsgrad Kontrolle}} \times 100$$

Die Ergebnisse gehen aus Tabelle B hervor.

TABELLE B

Wirkung gegen Erysiphe graminis an Gerste
Wirkstoffkonzentration: 10 mg/l

| Verbindung Nr. | Wirkungsgrad in Prozent |
|---|---|
| N-Methyl-N-tridecyl-2,6-dimethyl morpholinium-methosulfat (bekannt aus DE—PS 11 67 588) | 82 |
| Triforine (bekannt) | 86 |
| 7 | 87 |
| 32 | 94 |
| 75 | 82 |
| 94 | 92 |
| 130 | 96 |

Beispiel C

Getreiderost-Test (Puccinia recondita/Weizen)

In Töpfen angezogene Zeizenpflanzen der Sorte "Alcedo" werden im Zweiblattstadium auf die Höhe von 12 cm zurückgeschnitten. Das Sekundärblatt der Pflanzen wird entfernt. Danach werden die weizenpflanzen mit Wirkstoffzubereitungen besprüht, die aus 20 Gewichtsteilen Wirkstoff, 10 Gewichtsteilen Polyoxyethylensorbitanmonostearat (Tween 60), 5 Gewichtsteilen Polypropylenglykol, 25 Gewichtsteilen Cyclohexanon und 40 Gewichtsteilen Toluen hergestellt und mit Wasser auf die gewünschte Wirkstoffkonzentration verdünnt werden. Nach dem Antrocknen ds Spritzbelages werden die Pflanzen mit Sporen des Weizenbraunrostes (Puccinia recondita), die als Aufschwemmung in Wasser mit Tween 60-Zusatz appliziert werden, inokuliert. Anschließend werden die Versuchspflanzen für einen Zeitraum von 24 Stunden in einer Wasserdampf-gesättigten Inkubationskabine und danach bei Temperaturen von 20 bis 22°C und bei 70 bis 80% relativer Luftfeuchtigkeit im Gewächshaus aufgestellt.

Nach 10 Tagen wird der Rostbefall der Weizenpflanzen ermittelt.

Der Wirkungsgrad der Mittel wird nach der in Beispiel B beschriebenen Methode berechnet.

Die Ergebnisse sind in Tabelle C aufgeführt.

TABELLE C

Wirkung gegen Puccinia recondita an Weizen
Wirkstoffkonzentration: 500 mg/l

| Verbindung Nr. | Wirkungsgrad in Prozent |
|---|---|
| Triforine (bekannt) | 64 |
| 79 | 83 |
| 80 | 85 |
| 86 | 76 |

Beispiel D

Botrytis-Test (Botrytis cinerea/Ackerbohne (Vicia faba)-Blattfieder

Abgeschnittene Blattfieder von in Töpfen angezogenen Ackerbohnepflanzen (Vicia faba) der Sorte "Fribo" im Vierblattstadium werden mit Wirkstoffzubereitungen bestrichen, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach Antrocknen des Spritzbelages werden die Blätter mit einer Konidiensuspension von Botrytis cinerea inokuliert, die durch Abschwemmen von 12 bis 16 Tage alten Pilzkulturen auf Malzagar-Nährmedium (2% Malz) gewonnen wird. Die Ackerbohne-Blattfieder werden in einer Inkubationskabine in Schalen bei einer Temperatur von 22°C und 90 bis 100% relativer Luftfeuchtigkeit gehalten.

Nach 4 Tagen wird der Botrytis-Befall der Ackerbohne-Blattfieder bestimmt. Der auf die gesamte Blattfläche bezogene prozentuale Befall wird in den Wirkungsgrad der Mittel nach ABBOTT umgerechnet.

Die Ergebnisse gehen aus Tabelle D hervor.

TABELLE D

Wirkung gegen Botrytis cinerea auf Ackerbohne (Vicia faba)-Blattfieder
Wirkstoffkonzentration: 100 mg/l

| Verbindung Nr. | Wirkungsgrad in Prozent |
|---|---|
| Tridemorph (bekannt) | 42 |
| 76 | 74 |
| 79 | 47 |
| 86 | 45 |

Beispiel E

Phytophthora-Test (Phytophthora infestans/Tomate)

In Töpfen angezogene Tomatenpflanzen der Sorte "Tamina" werden im Dreiblattstadium taufeucht mit Wirkstoffzubereitungen besprüht, die aus 1 Gewichtsteil Wirkstoff, 100 Gewichtsteilen Dimethylformamid und 0,25 Gewichtsteilen Alkylarylpolyglykolether hergestellt und mit Wasser auf die gewünschte Wirkstoffkonzentration verdünnt werden. Nach Antrocknen des Spritzbelages werden die Tomatenpflanzen mit einer wäßrigen Zoosporensuspension von Phytophthora infestans inokuliert. Anschließend werden die Versuchspflanzen in einer Inkubationskammer bei Temperaturen zwischen 18 und 20°C und 95 bis 100% relativer Luftfeuchtigkeit aufgestellt.

Nach 5 Tagen wird der Phytophthora-Befall der Tomatenpflanzen bestimmt. Die erhaltenen Boniturwerte werden in den Befallsgrad und den Wirkungsgrad der Mittel, wie im Beispiel B beschrieben, umgerechnet.

Die Ergebnisse sind in Tabelle E aufgeführt.

TABELLE E

Wirkung gegen Phytophthora infestans auf Tomaten

| Verbindung Nr. | Wirkstoffkonzentration in mg/l | Wirkungsgrad in Prozent |
|---|---|---|
| Zineb (bekannt) | 100 | 57 |
| | 200 | 61 |
| 79 | 100 | 60 |
| | 200 | 77 |

Beispiel F

Pflanzenwachstumsregulations-Test

Gurkenpflanzen der Sorte "Eva" werden in Töpfen in Humuserde, welche ausreichend mit Nährstoffen

versorgt ist, bis zu einer Wuchshöhe von 9 cm in Gewächschaus herangezogen. Pro Versuchsvariante werden 10 Pflanzen verwendet. Die Gurkenpflanzen werden mit Wirkstoffzubereitungen besprüht, die aus 1 Gewichtsteil Wirkstoffe, 100 Gewichtsteilen Dimethylformamid und 0,25 Gewichtsteilen Alkylarylpolyglykolether hergestellt und mit Wasser auf die gewünschte Wirkstoffkonzentration verdünnt werden.

Nach einer Wachstumszeit von 14 Tagen nach der Mittelapplikation werden an den behandelten Pflanzen und den unbehandelten Kontrollpflanzen Längenmessungen vorgenommen.

Die Ergebnisse gehen aus Tabelle F hervor.

TABELLE F

Wirkung auf das Längenwachstum von Gurkenpflanzen bei Blattbehandlung
Wirkstoffkonzentration: 1000 mg/l

| Verbindung Nr | Pflanzenhöhe in cm | relativ |
|---|---|---|
| unbehandelte Kontrolle | 26,5 | 100 |
| 79 | 21,0 | 79,5 |

**Patentansprüche**

1. N-Alkyl-2,6-dimethylmorpholinio-carbonsäuremid-Salze der allgemeinen Formel I,

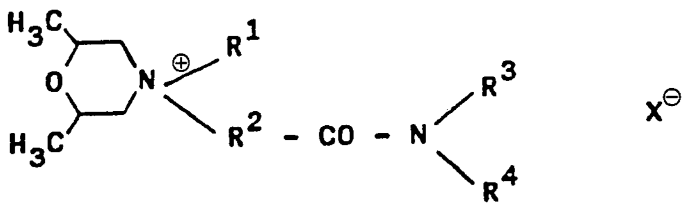 (I)

in der

$R^1$ geradkettiges oder verzweigtes Alkyl mit 6 bis 20 C-Atomen,

$R^2$ geradkettiges oder verzweigtes Alkylen mit 1 bis 6 C-Atomen,

$R^3$ und $R^4$ unabhängig voneinander gleich oder verschieden Wasserstoff, geradkettiges oder verzweigtes Alkyl mit 1 bis 20 C-Atomen, ein durch Hydroxy, Alkoxy mit 1 bis 4 C-Atomen, Cyano oder Dialkylamino mit 2 bis 16 C-Atomen substituiertes geradkettiges oder verzweigtes Alkyl mit 1 bis 6 C-Atomen, ein gegebenenfalls durch Halogen substituiertes Alkenyl mit 3 bis 6 C-Atomen, Cycloalkyl mit 3 bis 7 C-Atomen, Aryl, welches einfach oder mehrfach, gleich oder verschieden durch geradkettiges oder verzweigtes Alkyl mit 1 bis 6 C-Atomen, Alkoxy mit 1 bis 4 C-Atomen, Aryl-nieder-alkyl mit 7 bis 12 C-Atomen, Cycloalkyl mit 3 bis 7 C-Atomen, Acyl mit 1 bis 4 C-Atomen, Aryl, Halogen, Halogenalkyl mit 1 bis 4 C-Atomen, Halogenalkoxy mit 1 bis 4 C-Atomen, Alkylthio mit 1 bis 4 C-Atomen, Halogenalkylthio mit 1 bis 4 C-Atomen, Nitro, Cyano, Thiocyanato, NHCOR', NHCONR'R'', COOR', CONR'R'', $SO_2R'$ und/oder $SO_2NR'R''$, wobei R' und R'' unabhängig voneinander für Wasserstoff, geradkettiges oder verzweigtes Alkyl mit 1 bis 6 C-Atomen, Cycloalkyl mit 3 bis 7 C-Atomen, Aryl oder Aryl-nieder-alkyl mit 7 bis 12 C-Atomen steht, substituiert sein kann, Aryl-nieder-alkyl mit 7 bis 12 C-Atomen, welches einfach oder mehrfach, gleich oder verschieden durch geradkettiges oder verzweigtes Alkyl mit 1 bis 6 C-Atomen, Alkoxy mit 1 bis 4 C-Atomen, Acyl mit 1 bis 4 C-Atomen, Halogen, Halogenalkyl mit 1 bis 4 C-Atomen, Nitro und/oder Cyano substituiert sein kann,

$R^3$ und $R^4$ zusammen mit dem Stickstoffatom einen gegebenenfalls ein- oder zweifach durch Alkyl mit 1 bis 4 C-Atomen und/oder Halogen substituierten 3 fünf- oder sechsgliedrigen heterocyclischen Ring darstellt, der gegebenenfalls noch ein oder zwei weitere Heteroatome enthält, und

$X^-$ das Anion einer nicht phytotoxischen Säure bedeuten.

2. Verbindungen gemäß Anspruch 1, dadurch gekennzeichnet, daß $R^3$ Wasserstoff und $R^4$ Halogenphenyl bedeuten.

3. Verfahren zur Herstellung von N-Alkyl-2,6-dimethylmorpholiniocarbonsäureamid-Salzen der allgemeinen Formel I gemäß Anspruch 1, dadurch gekennzeichnet, daß man N-Alkyl-2,6-dimethylmorpholin der Formel II,

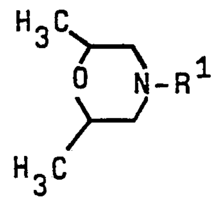

(II),

in der $R^1$ die im Anspruch 1 angegebene Bedeutung hat, mit einer Verbindung der Formel III,

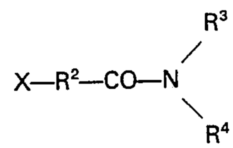

(III)

in der $R^2$, $R^3$ und $R^4$ die im Anspruch 1 angegebene Bedeutung haben und X für Halogen steht, gegebenenfalls in Gegenwart eines Verdünnungsmittels umsetzt.

4. Verfahren zur Herstellung von N-Alkyl-2,6-dimethylmorpholiniocarbonsäureamid-Salzen der allgemeinen Formel 1 gemäß Anspruch 1, dadurch gekennzeichnet, daß man ein 2,6-Dimethylmorpholino-carbonsäureamid der Formel IV,

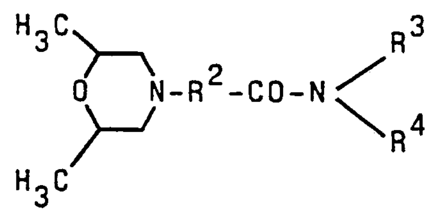

(IV),

in der $R^2$, $R^3$ und $R^4$ die im Anspruch 1 angegebene Bedeutung haben, mit einer Verbindung der Formel V,

$$R^1—X \qquad (V)$$

in der $R^1$ die in Anspruch 1 angegebene Bedeutung hat und X für Halogen steht, gegebenenfalls in Gegenwart eines Verdünnungsmittels umsetzt.

5. Fungizide Mittel mit zusätzlicher pflanzenwachstumsregulierender Wirkung enthaltend mindestens ein N-Alkyl-2,6-dimethylmorpholinio-carbonsäureamid-Salz der allgemeinen Formel I gemäß Anspruch und inerte Zusatzstoffe.

6. Verfahren zur Bekämpfung von Pilzen, dadurch gekennzeichnet, daß man mindestens ein N-Alkyl-2,6-dimethylmorpholino-carbonsäureamid-Salz der allgemeinen Formel I gemäß Anspruch 1 auf Pilze oder die vor Pilzebefall zu schützenden Gegenstände einwirken läßt.

7. Verwendung von N-Alkyl-2,6-dimethylmorpholino-carbonsäureamid-Salz der allgemeinen Formel I gemäß Anspruch 1 zur Bekämpfung von Pilzen und/oder zur Regulierung des Pflanzenwachstums.

8. N-iso-Tridecyl-2,6-dimethylmorpholinio-essigsäure-4-chloranilid-chlorid.

9. N-iso-Tridecyl-2,6-dimethylmorpholinio-essigsäure-3,5-dichloranilid-chlorid.

10. N-iso-Tridecyl-2,6-dimethylmorpholinio-essigsäure-3,5-dibromanilid-chlorid.

11. N-iso-Tridecyl-2,6-dimethylmorpholinio-essigsäure-aziridin-1-ylid-chlorid.

**Revendications**

1. Sels d'amides d'acides N-alkyl-2,6 diméthylmorpholinio-carboxyliques de formule générale I

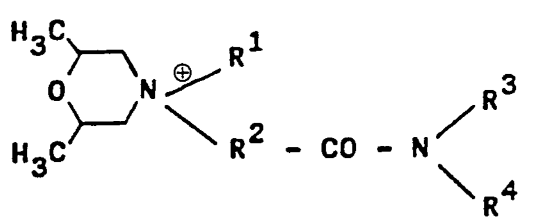

$X^{\ominus}$ (I)

dans laquelle

$R^1$ représente alkyle à 6 à 20 atomes C, à chaîne droite ou ramifiée

$R^2$, alkylène à 1 à 6 atomes C, à chaîne droite ou ramifiée

$R^3$ et $R^4$, identiques ou différents, indépendamment l'un de l'autre, hydrogène, alkyle à 1 à 20 atomes C, à chaîne droite ou ramifiée, un alkyle de 1 à 6 atomes C à chaîne droite ou ramifié éventuellement substituée par hydroxy, alcoxy à 1 à 4 atomes C, cyano ou dialkylamino à 2 à 16 atomes C, un alcényle à 3 à 6 atomes C, éventuellement substitué par halogène, cycloalkyle à 3 à 7 atomes C, aryle, qui peut être substitué une fois ou plusieurs fois, de façon identique ou différente, par alkyle à 1 à 6 atomes C, à chaîne droite ou ramifiée, alcoxy à 1 à 4 atomes C, aryle-alkyle inférieur à 7 à 12 atomes C, cycloalkyle à 3 à 7 atomes C, acyle à 1 à 4 atomes C, aryle, halogène, halogénalkyle à 1 à 4 atomes C, halogénalcoxy à 1 à 4 atomes C, alkylthio à 1 à 4 atomes C, halogénalkylthio à 1 à 4 atomes C, nitro, cyano, thiocyanato, NHCOR', HHCONR'R'', COOR', CONR'R'', $SO_2R'$ et/ou $SO_2NR'R''$, R' et R'' étant mis, indépendamment l'un de l'autre, pour hydrogène, alkyle à 1 à 6 atomes C, à chaîne droite ou ramifiée, cycloalkyle à 3 à 7 atomes C, aryle ou aryl-alkyle inférieur à 7 à 12 atomes C. aryl-alkyle inférieur à 7 à 12 atomes C, qui peut être substitué une fois ou plusieurs, de façon identique ou différente, par alkyle à 1 à 6 atomes C, à chaîne droite ou ramifiée, alcoxy à 1 à 4 atomes C, acyle à 1 à 4 atomes C, halogène, halogénalkyle à 1 à 4 atomes C, nitro et/ou cyano

$R^3$ et $R^4$ représentent, avec l'atome d'azote, un noyau hétérocyclique à cinq à six chaînons, éventuellement substitué une fois ou deux fois, par alkyle à 1 à 4 atomes C et/ou halogène, noyau qui contient éventuellement encore un ou deux autres hétéroatomes, et

$X^-$ représente l'anion d'un acide non phytotoxique.

2. Composés selon la revendication 1, caractérisé par le fait que $R^3$ représente hydrogène et $R^4$, halogénophényle.

3. Procédé de préparation de sels d'amides d'acides N-alkyl-2,6 diméthyl-morpholinio-carboxyliques de formule générale I, selon la revendication 1, caractérisé par le fait que l'on fait réagir, éventuellement en présence d'un diluant, de la N-alkyl-2,6-diméthylmorphiline de formule II

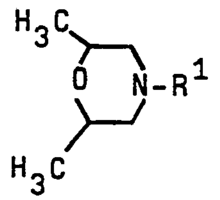

(II),

dans laquelle $R^1$ a la signification donnée dans la revendication 1, avec un composé de formule III

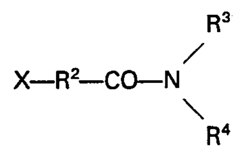

(III)

dans laquelle $R^2$, $R^3$ et $R^4$ ont les significations données dans la revendication 1 et X est mis pour halogène.

4. Procédé de préparation de sels d'amides d'acides N-alkyl-2,6 diméthyl-morpholinio-carboxyliques de formule générale I selon la revendication 1, caractérisé par le fait que l'on fait réagir, éventuellement en présence d'un diluant, un amide d'acide 2,6-diméthylmorpholinocarboxylique, de formule IV

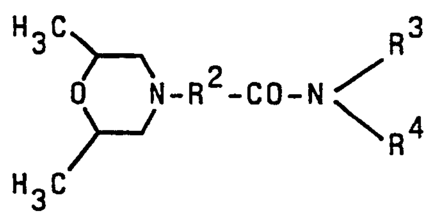

(IV),

dans laquelle $R^2$, $R^3$ et $R^4$ ont les significations données dans la revendication 1, avec un composé de formule V

$$R^1—X \qquad (V)$$

dans laquelle $R^1$ a la signification donnée dans la revendication 1 et X est mis pour halogène.

5. Agent fongicide à effet régulateur de la croissance des plantes additionnel, contenant au moins un sel d'amide d'acide N-alkyl-2,6 diméthyl-morpholinio-carboxylique de formule général I selon la revendication 1 et des additifs inertes.

6. Procédé de lutte contre les champignons, caractérisé par le fait que l'on fait agir sur les champignons

ou les objets à protéger contre l'attaque des champignons au moins un sel d'amide d'acide N-alkyl-2,6 diméthyl-morpholiniocarboxylique de formule générale I selon la revendication 1.

7. Utilisation d'un sel d'amide d'acide N-alkyl-2,6 diméthyl-morpholinio-carboxylique de formule générale I selon la revendication 1 pour la lutte contre les champignons et/ou la régularisation de la croissance des plantes.

8. 4-chloranilide-chlorure d'acide N-iso-tridecyl-2,6-diméthylmorpholinio-acétique.

9. 3,5-dichloranilide-chlorure d'acide N-iso-tridécyl-2,6-diméthylmorpholinio-acétique.

10. 3,5-dibromanilide-chlorure d'acide N-iso-tridécyl-2,6-diméthylmorpholinio-acétique.

11. Aziridin-1-ylide-chlorure d'acide N-iso-tridécyl-2,6-diméthylmorpholinio-acétique.

**Claims**

1. An N-alkyl-2,6-dimethylmorpholinocarboxamide salt of the formula I

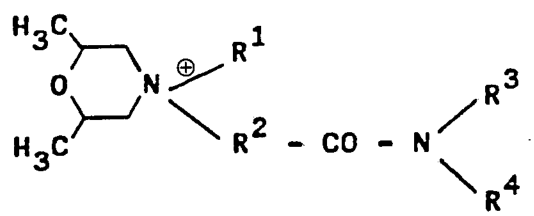

$X^{\ominus}$ (I)

where $R^1$ is straight-chain or branched alkyl of 6 to 20 carbon atoms, $R^2$ is straight-chain or branched alkylene of 1 to 6 carbon atoms, $R^3$ and $R^4$ are identical or different and independently of one another are each hydrogen, straight-chain or branched alkyl of 1 to 20 carbon atoms, or straight-chain or branched alkyl of 1 to 6 carbon atoms which is substituted by hydroxyl, alkoxy of 1 to 4 carbon atoms, cyano or dialkylamino of 2 to 16 carbon atoms, or are each unsubstituted or halogen-substituted alkenyl of 3 to 6 carbon atoms, cycloalkyl of 3 to 7 carbon atoms, aryl which is monosubstituted or polysubstituted by identical or different substituents from the group consisting of straight-chain or branched alkyl of 1 to 6 carbon atoms, alkoxy of 1 to 4 carbon atoms, aryl-(lower alkyl) of 7 to 12 carbon atoms, cycloalkyl of 3 to 7 carbon atoms, acyl of 1 to 4 carbon atoms, aryl, halogen, haloalkyl of 1 to 4 carbon atoms, haloalkoxy of 1 to 4 carbon atoms, alkylthio of 1 to 4 carbon atoms, haloalkylthio of 1 to 4 carbon atoms, nitro, cyano, thiocyanato, NHCOR', NHCONR'R'', COOR', CONR'R'', $SO_2R'$ and/or $SO_2NR'R''$, where R' and R'' independently of one another are each hydrogen, straight-chain or branched alkyl of 1 to 6 carbon atoms, cycloalkyl of 3 to 7 carbon atoms, aryl or aryl-(lower alkyl) of 7 to 12 carbon atoms, or are each aryl-(lower alkyl) of 7 to 12 carbon atoms which is monosubstituted or polysubstituted by identical or different substituents from the group consisting of straight-chain or branched alkyl of 1 to 6 carbon atoms, alkoxy of 1 to 4 carbon atoms, acyl of 1 to 4 carbon atoms, halogen, haloalkyl of 1 to 4 carbon atoms, nitro and/or cyano, $R^3$ and $R^4$ together with the nitrogen atom form a five-membered or six-membered heterocyclic ring which is unsubstituted or monosubstituted or disubstituted by alkyl of 1 to 4 carbon atoms and/or halogen, and may or may not contain one or two further hetero atoms, and $X^-$ is an anion of a non-phytotoxic acid.

2. A compound as claimed in claim 1, where $R^3$ is hydrogen and $R^4$ is halophenyl.

3. A process for manufacturing N-alkyl-2,6-dimethylmorpholinocarboxamide salts of the formula I as claimed in claim 1, wheein an N-alkyl-2,6-dimethylmorpholine of the formula II

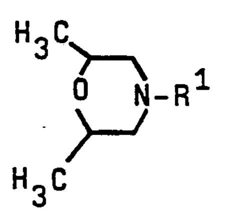

(II),

wherein $R^1$ has the meanings given in claim 1, is reacted with a compound of the formula III

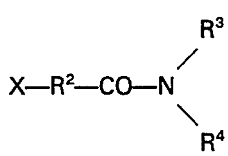

(III),

where $R^2$, $R^3$ and $R^4$ have the meanings given in claim 1 and X is halogen, in the presence or absence of a diluent.

4. A process for manufacturing N-alkyl-2,6-dimethylmorpholinocarboxamide salts of the formula I as claimed in claim 1, wherein a 2,6-dimethylmorpholinocarboxamide of the formula IV

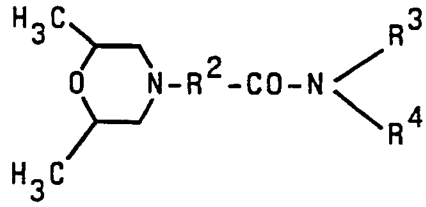

(IV),

where $R^2$, $R^3$ and $R^4$ have the meanings given in claim 1, is reacted with a compound of the formula V

$$R^1—X \qquad (V),$$

where $R^1$ has the meanings given in claim 1 and X is halogen, in the presence or absence of a diluent.

5. A fungicidal composition additionally possessing a plant growth-regulating action, which contains one or more N-alkyl-2,6-dimethylmorpholinocarboxamide salts of the formula I as claimed in claim 1 and an inert additive.

6. A method for controlling fungi, wherein one or more N-alkyl-2,6-dimethylmorpholinocarboxamide salts of the formula I as claimed in claim 1 are allowed to act on fungi or the objects to be protected against fungal attack.

7. The use of N-alkyl-2,6-dimethylmorpholinocarboxamide salts of the formula I as claimed in claim 1 for combating fungi and/or regulating plant growth.

8. 2-(N-Isotridecyl-2,6-dimethylmorpholino)-acetic acid 4-chloroanilide chloride.

9. 2-(N-Isotridecyl-2,6-dimethylmorpholino)-acetic acid 3,5-dichloranilide chloride.

10. 2-(N-Isotridecyl-2,6-dimethylmorpholino)-acetic acid 3,5-dibromoanilide chloride.

11. 2-(N-Isotridecyl-2,6-dimethylmorpholino)-acetic acid aziridin-1-ylide chloride.